Europäisches Patentamt
European Patent Office
Office européen des brevets

(19)

(11) Veröffentlichungsnummer: **0 443 127 A1**

(12)

# EUROPÄISCHE PATENTANMELDUNG

(21) Anmeldenummer: **90123546.5**

(22) Anmeldetag: **07.12.90**

(51) Int. Cl.5: **C07C 313/34**, C07C 311/54, A01N 47/34

(30) Priorität: **17.02.90 DE 4005116**

(43) Veröffentlichungstag der Anmeldung: **28.08.91 Patentblatt 91/35**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Maurer, Fritz, Dr., Nihon Tokushu Noyaku Seizo KK.**
**Yuki Research Center, 9511-4 Yuki**
**Yuki-shi, Ibaraki 307(JP)**
Erfinder: **Dutzmann, Stefan, Dr.**
**Kosenberg 10**
**W-4010 Hilden 1(DE)**
Erfinder: **Paulus, Wilfried, Dr.**
**Deswatinesstrasse 90**
**W-4150 Krefeld 1(DE)**

(54) **N-Sulfenyl-N-sulfonyl-Harnstoff-Derivate.**

(57) Beschrieben werden neue N-Sulfenyl-N'-sulfonyl-Harnstoff-Derivate der Formel (I)

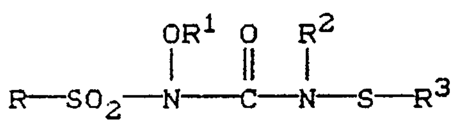

(I)

in welcher
R für Alkyl, Aryl oder Hetaryl steht,
$R^1$ für Alkyl steht,
$R^2$ für Alkyl oder Aryl steht und
$R^3$ für Halogenalkyl steht.

Beschrieben werden ferner neue Zwischenprodukte sowie mehrere Verfahren zur Herstellung der Verbindungen der Formel (I).

Die Verbindungen der Formel (I) können zur Bekämpfung von Schädlingen eingesetzt werden.

EP 0 443 127 A1

N-Sulfenyl-N'-sulfonyl-Harnstoff-Derivate

Die Erfindung betrifft neue N-Sulfenyl-N'-sulfonyl-Harnstoff-Derivate, neue Zwischenprodukte sowie mehrere Verfahren zu ihrer Herstellung und ihre Verwendung als Schädlingsbekämpfungsmittel und im Materialschutz.

Es ist bekannt, daß bestimmte N-Halogenalkyl-sulfenylcarbamate, wie beispielsweise der N-Methyl-N-dichlorfluormethylsulfenylcarbamidsäure-cyanmethylester oder der N-Methyl-N-dichlorfluormethylsulfenylcarbamidsäure-O-(1-cyan-2-methyl)-propylester fungizide Eigenschaften besitzen (vergleiche DE-OS 35 16 426).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue N-Sulfenyl-N'-sulfonyl-Harnstoff-Derivate der allgemeinen Formel (I) gefunden,

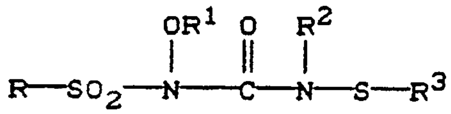

(I)

in welcher

R für Alkyl, Aryl oder Hetaryl steht,
$R^1$ für Alkyl steht,
$R^2$ für Alkyl oder Aryl steht und
$R^3$ für Halogenalkyl steht.

Weiterhin wurde gefunden, daß man die neuen N-Sulfenyl-N'-sulfonyl-Harnstoff-Derivate der allgemeinen Formel (I)

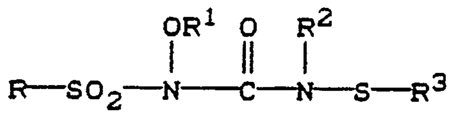

(I)

in welcher
R, $R^1$, $R^2$ und $R^3$ die oben angegebene Bedeutung haben,
erhält, wenn man

a) substituierte Sulfonamide der Formel (II)

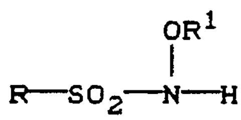

(II)

in welcher
R und $R^1$ die oben angegebene Bedeutung haben
mit N-Sulfenylcarbamidsäurehalogeniden der Formel (III)

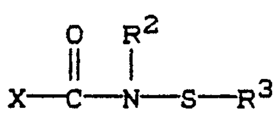

(III)

in welcher
$R^2$ und $R^3$ die oben angegebene Bedeutung haben
und

X für Halogen, insbesondere für Fluor, Chlor oder Brom steht,

gegebenenfalls in Gegenwart eines Verdünnungsmittels und gegebenenfalls in Gegenwart eines Säurebindemittels umsetzt, oder

b) Sulfonylharnstoff-Derivate der Formel (IV)

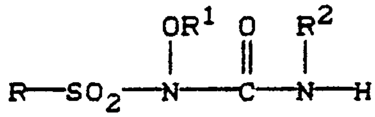

(IV)

in welcher

R, $R^1$ und $R^2$ die oben angegebene Bedeutung haben,

mit Schwefelhalogen-Verbindungen der Formel (V)

$$X-S-R^3 \qquad (V)$$

in welcher

$R^3$ die oben angegebene Bedeutung hat,
und

X für Halogen, insbesondere für Fluor, Chlor oder Brom steht,

gegebenenfalls in Gegenwart eines Verdünnungsmittels und gegebenenfalls in Gegenwart eines Säurebindemittels umsetzt, oder

c) Sulfonylhalogen-Verbindungen der Formel (VI)

$$R-SO_2-X \qquad (VI)$$

in welcher

R die oben angegebene Bedeutung hat,
und
X für Halogen, insbesondere für Fluor, Chlor oder Brom steht,

mit Sulfenylharnstoff-Derivaten der Formel (VII)

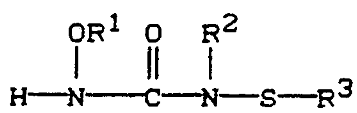

(VII)

in welcher

$R^1$, $R^2$ und $R^3$ die oben angegebene Bedeutung haben,
und

X für Halogen, insbesondere für Fluor, Chlor oder Brom steht,

gegebenenfalls in Gegenwart eines Verdünnungsmittels und gegebenenfalls in Gegenwart eines Säurebindemittels umsetzt.

Schließlich wurde gefunden, daß die neuen N-Sulfenyl-N'-sulfonyl-Harnstoff-Derivate der allgemeinen Formel (I) eine gute Wirkung gegen Schädlinge besitzen und auch im Materialschutz eingesetzt werden können.

Überraschenderweise zeigen die erfindungsgemäßen N-Sulfenyl-N'-sulfonyl-Harnstoff-Derivate der allgemeinen Formel (I) eine erheblich bessere fungizide Wirksamkeit als der aus dem Stand der Technik

bekannte N-Methyl-N-dichlorfluormethylsulfenylcarbamidsäure-cyanmethylester oder der N-Methyl-N-dichlorfluormethylsulfenylcarbamidsäure-O-(1-cyan-2-methyl)-propylester (DE-OS 35 16 426).

Bevorzugte Substituenten bzw. Bereiche der in den oben und nachstehend erwähnten Formeln aufgeführten Reste werden im folgenden erläutert:

Alkyl in den Definitionen von R, $R^1$ und $R^2$ in den allgemeinen Formeln, steht für gegebenenfalls substituiertes, geradkettiges oder verzweigtes Alkyl mit 1 bis 8 Kohlenstoffatomen, bevorzugt mit 1 bis 6 und besonders bevorzugt mit 1 bis 4 Kohlenstoffatomen; beispielhaft seien genannt Methyl, Ethyl, n- und i-Propyl, n-, i-, s- und t-Butyl.

Halogenalkyl steht in der Definition von $R^3$ in den allgemeinen Formeln für geradkettiges oder verzweigtes Halogenalkyl mit 1 bis 6 Kohlenstoffatomen, bevorzugt 1 bis 4 und besonders bevorzugt mit 1 oder 2 Kohlenstoffatomen und jeweils 1 bis 13, vorzugsweise 1 bis 9 und besonders bevorzugt 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl und Fluorbutyl.

Aryl in der Definition von R und $R^2$ in den allgemeinen Formeln steht für gegebenenfalls substituiertes Aryl mit vorzugsweise 6 bis 10 Kohlenstoffatomen im Arylteil. Beispielhaft und vorzugsweise seien unsubstituiertes oder substituiertes Phenyl oder Naphthyl, insbesondere Phenyl genannt.

Hetaryl in der Definition von R steht im allgemeinen für einen gegebenenfalls substituierten 5- bis 6-gliedrigen Ring, der 1 bis 3, bevorzugt 1 oder 2 gleiche oder verschiedene Heteroatome enthält Als Heteroatome seien vorzugsweise Sauerstoff, Schwefel und Stickstoff genannt; beispielhaft seien genannt: Pyrimidinyl, Pyrrolyl, Imidazol, Isothiazolyl, Oxazolyl, Pyridyl, Thienyl, Furyl, Pyridazinyl, Pyrazinyl, Isoxazolyl, Thiazolyl und Pyrazolyl.

Halogen steht im allgemeinen als Substituent oder in dem Rest Halogenalkyl für Fluor, Chlor, Brom und Iod, insbesondere für Fluor, Chlor und Brom.

Als Substituenten für die Hetaryl- und Arylreste seien vorzugsweise genannt:

Halogen; geradkettiges oder verzweigtes Alkyl mit 1 bis 6, bevorzugt mit 1 bis 4 Kohlenstoffatomen und besonders bevorzugt Methyl und Ethyl (die beispielhafte Aufzählung entspricht der weiter oben gegebenen); geradkettiges oder verzweigtes Alkoxy mit 1 bis 6, bevorzugt mit 1 bis 4 Kohlenstoffatomen und besonders bevorzugt mit 1 oder 2 Kohlenstoffatomen (beispielhaft seien genannt:

Methoxy, Ethoxy, n- und i-Propoxy, n-, i-, s- und t-Butoxy);

geradkettiges oder verzweigtes Halogenalkyl oder Halogenalkoxy mit vorzugsweise je 1 bis 4 Kohlenstoffatomen, besonders bevorzugt mit jeweils 1 oder 2 Kohlenstoffatomen und jeweils 1 bis 9, vorzugsweise 1 bis 5, gleichen oder verschiedenen Halogenatomen wie unter Halogen definiert (beispielhaft seien genannt: Fluormethyl, Chlormethyl, Brommethyl, Fluorethyl, Chlorethyl, Brommethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy); geradkettiges oder verzweigtes Alkoxycarbonyl mit vorzugsweise 1 bis 4 Kohlenstoffatomen im Alkoxyteil, besonders bevorzugt mit 1 oder 2 Kohlenstoffatomen im Alkoxyteil (beispielhaft seien genannt: Methoxycarbonyl, Ethoxycarbonyl, n-Propoxycarbonyl, i-Propoxycarbonyl, n-, i-, s- und t-Butoxycarbonyl); Nitro.

Die hier aufgeführten Definitionen gelten in entsprechender Weise auch für die im folgenden aufgeführten bevorzugten Kombinationen von Resten.

Die erfindungsgemäßen neuen N-Sulfenyl-N'-sulfonyl-Harnstoff-Derivate sind durch die Formel (I) allgemein definiert. Bevorzugt sind Verbindungen der Formel (I), bei welchen

R für geradkettiges oder verzweigtes Alkyl mit 1 bis 6 Kohlenstoffatomen steht, welches gegebenenfalls einfach oder mehrfach, gleich oder verschieden durch Fluor, Chlor oder Brom substituiert ist; weiterhin für jeweils gegebenenfalls einfach bis dreifach, gleich oder verschieden substituiertes Phenyl oder Naphthyl oder für einen gegebenenfalls einfach bis dreifach, gleich oder verschieden substituierten 5- bis 6-gliedrigen ungesättigten heterocyclischen Ring steht, welcher 1 bis 3 gleiche oder verschiedenen Heteroatome aus der Reihe Sauerstoff, Schwefel, Stickstoff enthält, wobei als Aryl- bzw- Hetarylsubstituenten jeweils infrage kommen: Halogen, geradkettiges oder verzweigtes Alkyl mit 1 bis 4 Kohlenstoffatomen, geradkettiges oder verzweigtes Alkoxy mit 1 bis 4 Kohlenstoffatomen, jeweils geradkettiges oder verzweigtes Halogenalkyl oder Halogenalkoxy mit jeweils 1 bis 4 Kohlenstoffatomen und jeweils 1 bis 9 gleichen oder verschiedenen Fluor-, Chlor- oder Bromatomen sowie geradkettiges oder verzweigtes Alkoxycarbonyl mit 1 bis 4 Kohlenstoff-

atomen im Alkoxyteil,

$R^1$ für geradkettiges oder verzweigtes Alkyl mit 1 bis 6 Kohlenstoffatomen steht,

$R^2$ für geradkettiges oder verzweigtes Alkyl mit 1 bis 6 Kohlenstoffatomen, Phenyl oder Naphthyl steht und

$R^3$ für geradkettiges oder verzweigtes Halogenalkyl mit 1 bis 4 Kohlenstoffatomen und 1 bis 9 gleichen oder verschiedenen Fluor-, Chlor- oder Bromatomen steht.

Besonders bevorzugt sind Verbindungen der Formel (I), bei welchen

R für Methyl, Ethyl, n-Propyl, i-Propyl, für jeweils gegebenenfalls einfach oder zweifach, gleich oder verschieden substituiertes Phenyl, Pyrrolyl, Thienyl, Furyl, Pyrazolyl, Imidazolyl, Thiazolyl, Isothiazolyl oder Pyridyl steht, wobei als Substituenten jeweils infrage kommen: Fluor, Chlor, Brom, Methyl, Ethyl, Methoxy, Ethoxy, Trifluormethyl, Trichlormethyl, Dichlorfluormethyl, Chlordifluormethyl, Trifluormethoxy, Trichlormethoxy, Methoxycarbonyl und Ethoxycarbonyl,

$R^1$ für Methyl, Ethyl, n-Propyl oder i-Propyl steht,

$R^2$ für Methyl, Ethyl, n-Propyl, i-Propyl, n-Butyl, i-Butyl, s-Butyl, t-Butyl oder Phenyl steht und

$R^3$ für Trifluormethyl, Trichlormethyl, Dichlorfluormethyl, Chlordifluormethyl, Difluormethyl, Fluormethyl, Dichlormethyl, Chlormethyl, Chlorfluormethyl, 2,2,2-Trifluorethyl oder 2,2,2-Trichlorethyl steht.

Im einzelnen seien außer den bei den Herstellungsbeispielen genannten Verbindungen die folgenden N-Sulfenyl-N'-sulfonyl-Harnstoff-Derivate der Formel (I) genannt:

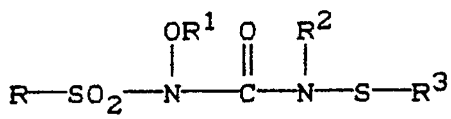

(I)

N-Dichlorfluormethylsulfenyl-N-methyl-N'-methoxy-N'-3-chlorphenyl-,-4-chlorphenyl-, -4-nitrophenyl-, 4-fluorphenyl-, -3-trifluormethylphenyl-, -3-trifluormethoxyphenyl-, -2-pyridinyl-, -3-pyridinyl-, -2-thienyl-sulfonylharnstoff; N-Dichlorfluormethyl-N-methyl-N'-4-methylphenylsulfonyl-N'-ethoxy-,-N'-propoxy-, -N'-isopropoxyharnstoff; N-Dichlorfluormethyl-N-methyl-, -N-ethyl-, -N-propyl-, -N-isopropyl-, -N-phenyl-N'-methoxy-N'-ethylsulfonylharnstoff; N-Dichlorfluormethyl-N-ethyl-N-propyl-, -N-isopropyl-, -N-phenyl-N'-methoxy-N'-4-methylphenylsulfonylharnstoff; N-Dichlorfluormethyl-N-methyl-, -N-ethyl-, -N-propyl-, -N-isopropyl-, -N-phenyl-N'-methoxy-N'-4-chlorphenylsulfonylharnstoff; N-Dichlorfluormethyl-N-ethyl-, -N-propyl-, -N-isopropyl-, -N- phenyl-N'-methoxy-N'-2-trifluormethoxyphenylsulfonylharnstoff; N-Chlordifluormethyl-N-methyl-N'-methoxy-N'-methylsulfonyl-, -N'-phenylsulfonyl-, -N'-4-methylphenylsulfonyl-, -N'-4-chlorphenylsulfonyl-, -N'-2-fluorphenylsulfonylharnstoff.

Verwendet man beispielsweise N-Methoxy-methansulfonsäureamid und N-Dichlorfluormethylsulfenyl-N-methylcarbamidsäurefluorid als Ausgangsstoffe und Kaliumfluorid als Säurebindemittel, so läßt sich der Reaktionsablauf des erfindungsgemäßen Verfahrens (a) durch das folgende Formelschema darstellen:

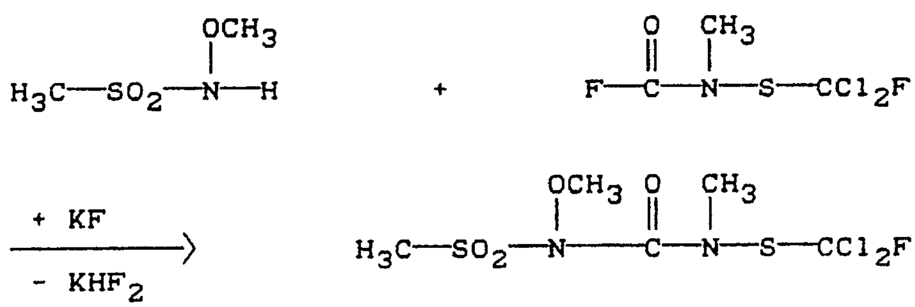


Verwendet man beispielsweise N-Ethoxy-N-methylsulfonyl-N'-methyl-Harnstoff und Dichlorfluormethylschwefelfluorid als Ausgangsstoffe und Kalium-tert-butylat als Säurebindemittel, so läßt sich der Reaktionsablauf des erfindungsgemäßen Verfahrens (b) durch das folgende Formelschema darstellen:

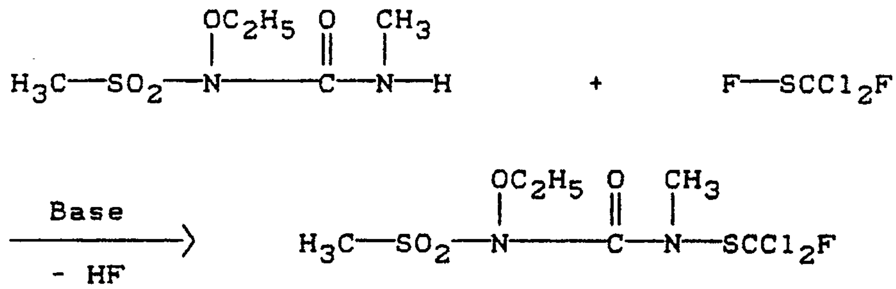


Verwendet man beispielsweise N-Dichlorfluormethylsulfenyl-N-isopropyl-N'-methoxy-Harnstoff und Methylsulfonylchlorid als Ausgangsstoff sowie Natrium-tert-amylat als Säurebindemittel, so läßt sich der Reaktionsablauf des erfindungsgemäßen Verfahrens (c) durch das folgende Formelschema darstellen:

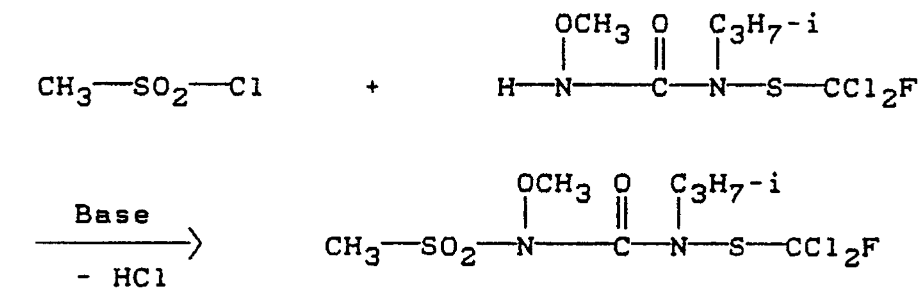


Die beim erfindungsgemäßen Verfahren (a) zur Herstellung von Verbindungen der Formel (I) als Ausgangsstoffe zu verwendenden substituierten Sulfonamide sind durch die Formel (II) allgemein definiert.

In Formel (II) haben R und $R^1$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R und R1 angegeben wurden.

Die substituierten Sulfonamide der Formel (II) sind bekannt und/oder können nach an sich bekannten Verfahren hergestellt werden (vergleiche z.B. Z. Naturforsch. 36 b, 1673 - 1674 (1981)).

Die beim erfindungsgemäßen Verfahren (a) zur Herstellung von Verbindungen der Formel (I) weiterhin als Ausgangsstoffe zu verwendenden N-Sulfenylcarbamidsäurehalogenide sind durch die Formel (III) allgemein definiert.

In Formel (III) haben $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und $R^3$ angegeben wurden, und X steht für Halogen, insbesondere für Fluor, Chlor oder Brom.

N-Sulfenylcarbamidsäurehalogenide der Formel (III) sind bekannt und/oder können nach bekannten Verfahren hergestellt werden (vergleiche z.B. US 4 013 774; DE-OS 2 362 686).

Die beim erfindungsgemäßen Verfahren (b) zur Herstellung von Verbindungen der Formel (I) als Ausgangsstoffe zu verwendenden Sulfonylharnstoff-Derivate sind durch die Formel (IV) allgemein definiert.

In Formel (IV) haben R, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R, $R^1$ und $R^2$ angegeben wurden.

Die Sulfonylharnstoff-Derivate der Formel (IV) sind neu und ebenfalls Gegenstand der vorliegenden Erfindung. Ausgenommen ist die Verbindung N-Methoxy-N-[(3,5-dinitro-4-diisopropylamino-phenyl)-sulfonyl]-N'-methyl-Harnstoff (DE-OS 2 227 744).

Man erhält die neuen Sulfonylharnstoff-Derivate der Formel (IV) indem man substituierte Sulfonamide der Formel (II)

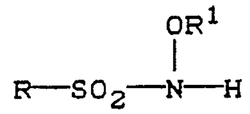

(II)

in welcher
R und $R^1$ die oben angegebene Bedeutung haben
mit Isocyanaten der Formel (VIII)

$$R^2—NCO \qquad (VIII)$$

in welcher

$R^2$ die oben angegebene Bedeutung hat,
oder mit Carbamidsäurehalogeniden der Formel (IX)

$$R^2—NH—CO—X \qquad (IX)$$

in welcher
$R^2$ die oben angegebene Bedeutung hat und
X für Fluor, Chlor oder Brom steht,
gegebenenfalls in Gegenwart eines Verdünnungsmittels wie beispielsweise Toluol, Tetrahydrofuran oder Dioxan und gegebenenfalls in Gegenwart eines Säurebindemittels wie beispielsweise Natriumcarbonat oder Kalium-tert-butylat bei Temperaturen zwischen 0 °C und 110 °C umsetzt.

Isocyanate der Formel (VIII) und Carbamidsäurehalogenide der Formel (IX) sind allgemein bekannte Verbindungen der organischen Chemie.

Die beim erfindungsgemäßen Verfahren (b) zur Herstellung von Verbindungen der Formel (I) außerdem als Ausgangsstoffe zu verwendenden Schwefelhalogen-Verbindungen sind durch die Formel (V) allgemein definiert.

In Formel (V) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde. X steht für Halogen, insbesondere für Fluor, Chlor oder Brom.

Schwefelhalogen-Verbindungen der Formel (V) sind allgemein bekannte Verbindungen der organischen Chemie.

Die beim erfindungsgemäßen Verfahren (c) zur Herstellung von Verbindungen der Formel (I) als Ausgangsstoffe zu verwendenden Sulfenylharnstoff-Derivate sind durch die Formel (VII) allgemein definiert.

In Formel (VII) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Sulfenylharnstoff-Derivate der Formel (VII) sind neu und ebenfalls Gegenstand der vorliegenden Erfindung. Man erhält die neuen Sulfenylharnstoff-Derivate der Formel (VII) indem man Hydroxylamine der Formel (X)

$$R^1O—NH_2 \qquad (X)$$

in welcher
$R^1$ die oben angegebene Bedeutung hat
mit N-Sulfenylcarbamidsäurehalogeniden der Formel (III)

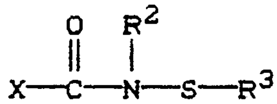

(III)

in welcher

$R^2$ und $R^3$ die oben angegebene Bedeutung haben und

X für Halogen, insbesondere für Fluor, Chlor oder Brom steht,

gegebenenfalls in Gegenwart eines Verdünnungsmittels wie beispielsweise Toluol, Tetrahydrofuran oder Dioxan und gegebenenfalls in Gegenwart eines Säurebindemittels wie beispielsweise Natriumcarbonat oder Kalium-tert-butylat bei Temperaturen zwischen 0 °C und 110 °C umsetzt.

Die beim erfindungsgemäßen Verfahren (c) zur Herstellung von Verbindungen der Formel (I) außerdem als Ausgangsstoffe zu verwendenden Sulfonylhalogen-Verbindungen sind durch die Formel (VI) allgemein definiert.

In Formel (VI) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde und X steht für Halogen, insbesondere für Fluor, Chlor oder Brom.

Sulfonylhalogen-Verbindungen der Formel (VI) sind allgemein bekannte Verbindungen der organischen Chemie.

Als Verdünnungsmittel zur Durchführung der erfindungsgemäßen Verfahren (a), (b) und (c) kommen vorzugsweise alle inerten organischen Lösungsmittel infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether, wie Diethylether, Diisopropylether, Dioxan und Tetrahydrofuran, Ketone, wie Aceton, Butanon, Methylisopropyl-und Methylisobutylketon, Nitrile, wie Acetonitril und Propionitril sowie die hochpolaren Lösungsmittel Dimethylsulfoxid und Hexamethylphosphorsäuretriamid.

Die erfindungsgemäßen Verfahren (a), (b) und (c) werden vorzugsweise in Gegenwart eines geeigneten Säurebindemittels durchgeführt. Als solche kommen alle üblicherweise verwendbaren anorganischen und organischen Basen infrage. Vorzugsweise verwendet man Alkalimetallalkoholate wie Natrium- oder Kalium-tert-butylat, Natrium-oder Kalium-tert-amylat, Alkalimetallcarbonate wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und 1,5-Diazabicyclo(4,3,0)-non-5-en. Falls es sich bei der zu bindenden Säure um Fluorwasserstoffsäure handelt, verwendet man vorzugsweise Kaliumfluorid als Säurebindemittel.

Die Reaktionstemperaturen können bei den erfindungsgemäßen Verfahren (a), (b) und (c) in einem größeren Bereich variiert werden. Im allgemeinen arbeitet man bei Temperaturen von 0 °C bis +100 °C, vorzugsweise bei Temperaturen von +10 °C bis +80 °C.

Die erfindungsgemäßen Verfahren (a), (b) und (c) werden im allgemeinen bei Normaldruck durchgeführt. Zur Durchführung der erfindungsgemäßen Verfahren (a), (b) und (c) setzt man die zu verwendenden Ausgangsstoffe im allgemeinen in angenähert äquimolaren Mengen ein. Ein Überschuß der einen oder anderen Komponente bis etwa 10 %. ist jedoch problemlos möglich.

Bei den erfindungsgemäßen Verfahren (a), (b) und (c) wird die Umsetzung vorzugsweise unter Verwendung eines der oben genannten Säurebindemittel in einem der oben angegebenen Verdünnungsmitteln durchgeführt. Das Reaktionsgemisch wird mehrere Stunden bei der erforderlichen Temperatur gerührt. Die Aufarbeitung der Reaktionsmischung und die Isolierung der erfindungsgemäßen Reaktionsprodukte der Formel (I) erfolgt in allgemein üblicher Art und Weise.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezahlten Oberbegriffe fallen, genannt:

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg bei der Saatgutbehandlung einsetzen, beispielsweise gegen Fusarium culmorum an Weizen, sowie weiterhin protektiv gegen Leptosphaeria nodonum an Weizen und protektiv gegen Cochliobolus sativus an Gerste.

Darüber hinaus zeigen die erfindungsgemäßen Wirkstoffe außerdem eine fungizide Wirkung gegen Fusarien, Pyricularia oryzae an Reis sowie eine breite und gute in vitro-Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt: Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,

Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Beim Einsatz im Materialschutz können die erfindungsgemäßen Wirkstoffe je nach Anwendungsgebiet in die üblichen Formulierungen überführt werden, wie Lösungen, Emulsionen, Suspensionen, Pulver, Pasten

und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihydroxy-5,5'-dichlor)-diphenylmethan und 3-Methyl-4-chlor-phenol, Organo-Zinnverbindungen, N-Trihalogenmethylthio-Verbindungen, wie Folpet, Fluorfolpet, Dichlofluanid.

Die Herstellung und die Verwendung der erfindungsgemäßen Wirkstoffe werden durch die folgenden Beispiele veranschaulicht.

## Herstellungsbeispiele

### Beispiel 1

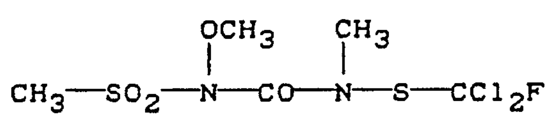

(Verfahren (a))

Zu einer Mischung aus 16,6 g (0,1 Mol) 80prozentigem N-Methoxy-methansulfonsäureamid, 6,77 g (0,12 Mol) Kaliumfluorid und 200 ml Acetonitril tropft man unter Kühlung bei ca. 5 °C 22,25 g (0,11 Mol) N-Dichlorfluormethylsulfenyl-N-methylcarbamidsäurefluorid. Man rührt das Reaktionsgemisch 18 Stunden bei Raumtemperatur nach, destilliert das Lösungsmittel im Vakuum ab und versetzt den Rückstand mit 100 ml Wasser und 100 ml Methylenchlorid. Nach Umschütteln wird das Wasser abgetrennt, die organische Phase schüttelt man mit 50 ml Wasser, trocknet sie über Natriumsulfat und filtriert sie über ca. 200 g Kieselgel. Das Filtrat wird unter vermindertem Druck eingeengt. Den Rückstand destilliert man bei 60 °C im Hochvakuum an. Man erhält so 22,9 g (70,7% der Theorie) N-Dichlorfluormethylsulfenyl-N-methyl-N'-methoxy-N'-methylsulfonylharnstoff vom Schmelzpunkt 48 °C.

Analog zum Herstellungsbeispiel 1 und gemäß den allgemeinen Angaben zu den erfindungsgemäßen Verfahren (a), (b) und (c) können die in Tabelle 1 aufgeführten Verbindungen der allgemeinen Formel (I) hergestellt werden.

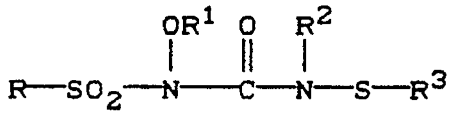

Tabelle 1

| Bsp. Nr. | R | $R^1$ | $R^2$ | $R^3$ | physikal. Daten |
|---|---|---|---|---|---|
| 2 | $-CH_3$ | $-C_2H_5$ | $-CH_3$ | $-CCl_2F$ | $n_D^{20}$ : 1,4944 |
| 3 | $-CH_3$ | $-CH_3$ | $-C_3H_7$-i | $-CCl_2F$ | Smp.: 65° C |
| 4 | 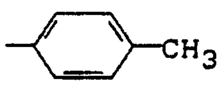 | $-CH_3$ | $-CH_3$ | $-CCl_2F$ | Smp.: 95° C |
| 5 | 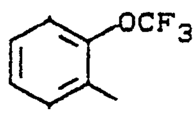 | $-CH_3$ | $-CH_3$ | $-CCl_2F$ | Smp.: 108° C |
| 6 | 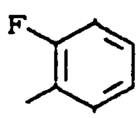 | $-CH_3$ | $-CH_3$ | $-CCl_2F$ | |
| 7 | 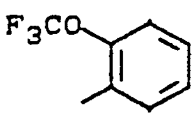 | $-CH_3$ | $-CH_3$ | $-CCl_3$ | |

Tabelle 1 (Fortsetzung)

| Bsp. Nr. | R | $R^1$ | $R^2$ | $R^3$ | physikal. Daten |
|---|---|---|---|---|---|
| 8 | 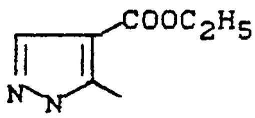 | $-CH_3$ | $-CH_3$ | $-CCl_2F$ | |
| 9 | 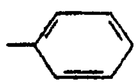 | $-CH_3$ | $-CH_3$ | $-CCl_2F$ | |
| 10 | $-C_2H_5$ | $-CH_3$ | $-CH_3$ | $-CCl_2F$ | |
| 11 | 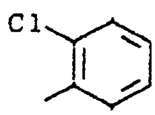 | $-CH_3$ | $-CH_3$ | $-CCl_2F$ | |
| 12 | 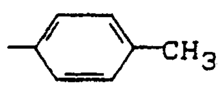 | $-C_3H_7$-n | $-CH_3$ | $-CCl_2F$ | |
| 13 | 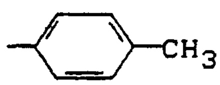 | $-CH_3$ | $-CH_3$ | $-CF_3$ | |
| 14 | $-CH_3$ | $-CH_3$ | $-CH_3$ | $-CClF_2$ | |
| 15 | 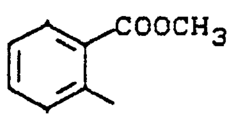 | $-CH_3$ | $-CH_3$ | $-CCl_2F$ | |

Anwendungsbeispiele

In den folgenden Anwendungsbeispielen wurden die nachstehend aufgeführten Verbindungen als Vergleichsubstanzen eingesetzt:

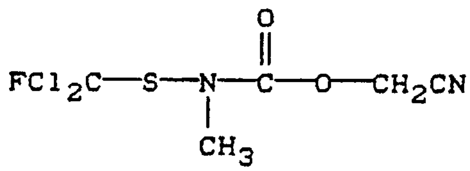

(A)

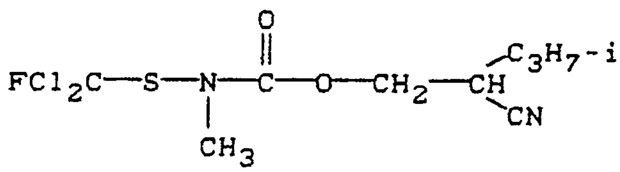

(B)

N-Methyl-N-dichlorfluormethylsulfenylcarbamidsäurecyanmethylester (A) und N-Methyl-N-dichlorfluormethylsulfenylcarbamidsäure-O-(1-cyan-2-methyl)-propylester (beide Verbindungen aus DE-OS 35 16 426).

Beispiel A

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca, 18 °C in Saatkästen, die täglich 15 Stunden dem Licht ausgesetzt werden.

Ca. 3 Wochen nach der Aussaat erfolgt die Auswertung der Pflanzen auf Symptome.

Eine deutliche Überlegenheit in der Wirksamkeit gegenüber dem Stand der Technik zeigt bei diesem Test z.B. die Verbindung des Herstellungsbeispiels (1).

Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensupension von Leptosphaeria nodorum besprüht. Die Pflanzen verbleiben 48 Stunden bei 20 °C und 100 %. relativer Luftfeuchtigkeit in einer Inkubationskabine.

Die Pflanzen werden in einem Gewächshaus bei einer Temperatur von ca. 15 °C und einer relativen Luftfeuchtigkeit con ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß Herstellungsbeispiele: (2), (3) und (4).

Beispiel C

Cochliobolus sativus-Test (Gerste) / protektiv

Lösungsmittel: 100 Gewichtsteile Dimethylformamid

Emulgator: 0,25 Gewichtsteile Alkylarylpolyglykolether

Zur Herstellung einre zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß Herstellungsbeispiele: (2) und (4).

## Patentansprüche

**1.** N-Sulfenyl-N'-sulfonyl-Harnstoff-Derivate der allgemeinen Formel (I),

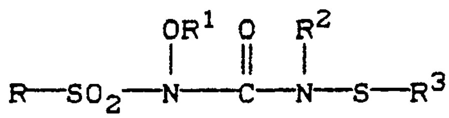

(I)

in welcher

- R für Alkyl, Aryl oder Hetaryl steht,
- $R^1$ für Alkyl steht,
- $R^2$ für Alkyl oder Aryl steht und
- $R^3$ für Halogenalkyl steht.

**2.** N-Sulfenyl-N'-sulfonyl-Harnstoff-Derivate der Formel (I), gemäß Anspruch 1, in welcher

- R für geradkettiges oder verzweigtes Alkyl mit 1 bis 6 Kohlenstoffatomen steht, welches gegebenenfalls einfach oder mehrfach, gleich oder verschieden durch Fluor, Chlor oder Brom substituiert ist; weiterhin für jeweils gegebenenfalls einfach bis dreifach, gleich oder verschieden substituiertes Phenyl oder Naphthyl oder für einen gegebenenfalls einfach bis dreifach, gleich oder verschieden substituierten 5- bis 6-gliedrigen ungesättigten heterocyclischen Ring steht, welcher 1 bis 3 gleiche oder verschiedenen Heteroatome aus der Reihe Sauerstoff, Schwefel, Stickstoff enthält, wobei als Aryl- bzw. Hetarylsubstituenten jeweils infrage kommen: Halogen, geradkettiges oder verzweigtes Alkyl mit 1 bis 4 Kohlenstoffatomen, geradkettiges oder verzweigtes Alkoxy mit 1 bis 4 Kohlenstoffatomen, jeweils geradkettiges oder verzweigtes Halogenalkyl oder Halogenalkoxy mit jeweils 1 bis 4 Kohlenstoffatomen und jeweils 1 bis 9 gleichen oder verschiedenen Fluor-, Chlor-oder Bromatomen sowie geradkettiges oder verzweigtes Alkoxycarbonyl mit 1 bis 4 Kohlenstoffatomen im Alkoxyteil,
- $R^1$ für geradkettiges oder verzweigtes Alkyl mit 1 bis 6 Kohlenstoffatomen steht,
- $R^2$ für geradkettiges oder verzweigtes Alkyl mit 1 bis 6 Kohlenstoffatomen, Phenyl oder Naphthyl steht und
- $R^3$ für geradkettiges oder verzweigtes Halogenalkyl mit 1 bis 4 Kohlenstoffatomen und 1 bis 9 gleichen oder verschiedenen Fluor-, Chlor- oder Bromatomen steht.

**3.** N-Sulfenyl-N'-sulfonyl-Harnstoff-Derivate der Formel (I), gemäß Anspruch 1, in welcher

- R für Methyl, Ethyl, n-Propyl, i-Propyl, für jeweils gegebenenfalls einfach oder zweifach, gleich oder verschieden substituiertes Phenyl, Pyrrolyl, Thienyl, Furyl, Pyrazolyl, Imidazolyl, Thiazolyl, Isothiazolyl oder Pyridyl steht, wobei als Substituenten jeweils infrage kommen: Fluor, Chlor, Brom, Methyl, Ethyl, Methoxy, Ethoxy, Trifluormethyl, Trichlormethyl, Dichlorfluormethyl, Chlordifluormethyl, Trifluormethoxy, Trichlormethoxy, Methoxycarbonyl und Ethoxycarbonyl,
- $R^1$ für Methyl, Ethyl, n-Propyl oder i-Propyl steht,

$R^2$ für Methyl, Ethyl, n-Propyl, i-Propyl, n-Butyl, i-Butyl, s-Butyl, t-Butyl oder Phenyl steht und
$R^3$ für Trifluormethyl, Trichlormethyl, Dichlorfluormethyl, Chlordifluormethyl, Difluormethyl, Fluormethyl, Dichlormethyl, Chlormethyl, Chlorfluormethyl, 2,2,2-Trifluorethyl oder 2,2,2-Trichlorethyl steht.

**4.** Verfahren zur Herstellung von N-Sulfenyl-N'-sulfonyl-Harnstoff-Derivaten der allgemeinen Formel (I)

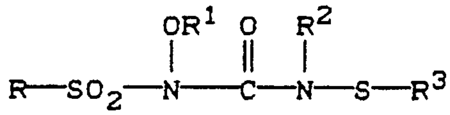

(I)

in welcher
R für Alkyl, Aryl oder Hetaryl steht,
$R^1$ für Alkyl steht,
$R^2$ für Alkyl oder Aryl steht und
$R^3$ für Halogenalkyl steht,
dadurch gekennzeichnet, daß man
a) substituierte Sulfonamide der Formel (II)

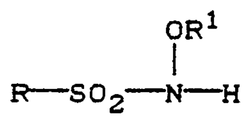

(II)

in welcher
R und $R^1$ die oben angegebene Bedeutung haben
mit N-Sulfenylcarbamidsäurehalogeniden der Formel (III)

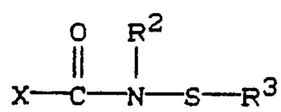

(III)

in welcher
$R^2$ und $R^3$ die oben angegebene Bedeutung haben
und

X für Halogen, insbesondere für Fluor, Chlor oder Brom steht,
gegebenenfalls in Gegenwart eines Verdünnungsmittels und gegebenenfalls in Gegenwart eines Säurebindemittels umsetzt, oder
b) Sulfonylharnstoff-Derivate der Formel (IV)

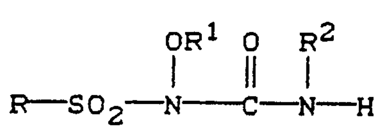

(IV)

in welcher
R, $R^1$ und $R^2$ die oben angegebene Bedeutung haben,
mit Schwefelhalogen-Verbindungen der Formel (V)

$$X—S—R^3 \qquad (V)$$

in welcher
$R^3$ die oben angegebene Bedeutung hat, und

X für Halogen, insbesondere für Fluor, Chlor oder Brom steht,

gegebenenfalls in Gegenwart eines Verdünnungsmittels und gegebenenfalls in Gegenwart eines Säurebindemittels umsetzt, oder

c) Sulfonylhalogen-Verbindungen der Formel (VI)

$$R—SO_2—X \qquad (VI)$$

in welcher

R die oben angegebene Bedeutung hat,
und
X für Halogen, insbesondere für Fluor, Chlor oder Brom steht,

mit Sulfenylharnstoff-Derivaten der Formel (VII)

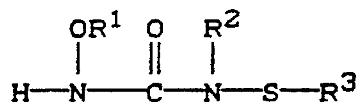

(VII)

in welcher
$R^1$, $R^2$ und $R^3$ die oben angegebene Bedeutung haben,
und

X für Halogen, insbesondere für Fluor, Chlor oder Brom steht,

gegebenenfalls in Gegenwart eines Verdünnungsmittels und gegebenenfalls in Gegenwart eines Säurebindemittels umsetzt.

**5.** Schädlingsbekämpfungsmittel, gekennzeichnet durch einen Gehalt an mindestens einem N-Sulfenyl-N'-sulfonyl-Harnstoff-Derivat der Formel (I) nach den Ansprüchen 1 bis 4.

**6.** Verwendung von N-Sulfenyl-N'-sulfonyl-Harnstoff-Derivaten der Formel (I) nach den Ansprüchen 1 bis 4 zur Bekämpfung von Schädlingen.

**7.** Verfahren zur Bekämpfung von Schädlingen, dadurch gekennzeichnet, daß man N-Sulfenyl-N'-sulfonyl-Harnstoff-Derivate der Formel (I) nach den Ansprüchen 1 bis 4 auf Schädlinge und/oder ihren Lebensraum einwirken läßt.

**8.** Verfahren zur Herstellung von Schädlingsbekämpfungsmitteln, dadurch gekennzeichnet, daß man N-Sulfenyl-N'-sulfonyl-Harnstoff-Derivate der Formel (I) nach den Ansprüchen 1 bis 4 mit Streckmitteln und/oder oberflächenaktiven Mitteln vermischt.

**9.** Sulfonylharnstoff-Derivate der Formel (IV)

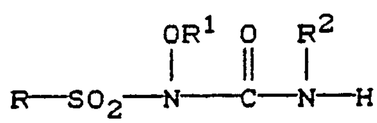

(IV)

in welcher
R für Alkyl, Aryl oder Hetaryl steht,
$R^1$ für Alkyl steht und
$R^2$ für Alkyl oder Aryl steht.

**10.** Verfahren zur Herstellung von Sulfonylharnstoff-Derivaten der Formel (IV), gemäß Anspruch 9, dadurch gekennzeichnet daß man substituierte Sulfonamide der Formel (II)

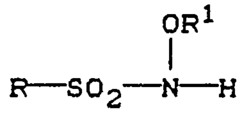

(II)

in welcher
R und $R^1$ die in Anspruch 9 angegebene Bedeutung haben
mit Isocyanaten der Formel (VIII)

$$R^2\text{—NCO} \qquad \text{(VIII)}$$

in welcher

$R^2$ die in Anspruch 9 angegebene Bedeutung hat,
oder mit Carbamidsäurehalogeniden der Formel (IX)

$$R^2\text{—NH—CO—X} \qquad \text{(IX)}$$

in welcher

$R^2$ die in Anspruch 9 angegebene Bedeutung hat und
X für Fluor, Chlor oder Brom steht,
gegebenenfalls in Gegenwart eines Verdünnungsmittels und gegebenenfalls in Gegenwart eines Säurebindemittels bei Temperaturen zwischen 0 °C und 110 °C umsetzt.

**11.** Sulfenylharnstoff-Derivate der Formel (VII)

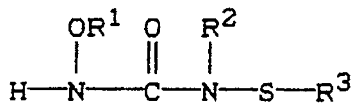

(VII)

in welcher

$R^1$ für Alkyl steht,
$R^2$ für Alkyl oder Aryl steht und
$R^3$ für Halogenalkyl steht.

**12.** Verfahren zur Herstellung von Sulfenylharnstoff-Derivaten der Formel (VII) gemäß Anspruch 11, dadurch gekennzeichnet, daß man Hydroxylamine der Formel (X)

$$R^1\text{O—NH}_2 \qquad \text{(X)}$$

in welcher

$R^1$ die in Anspruch 11 angegebene Bedeutung hat
mit N-Sulfenylcarbamidsäurehalogeniden der Formel (III)

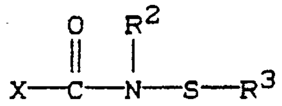

(III)

in welcher
$R^2$ und $R^3$ die in Anspruch 11 angegebene Bedeutung haben und
X für Halogen, insbesondere für Fluor, Chlor oder Brom steht,
gegebenenfalls in Gegenwart eines Verdünnungsmittels und gegebenenfalls in Gegenwart eines Säurebindemittels bei Temperaturen zwischen 0° C und 110° C umsetzt.

Europäisches Patentamt

# EUROPÄISCHER RECHERCHENBERICHT

Nummer der Anmeldung

EP 90123546.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | EP - A1 - 0 087 704 (BAYER) * Ansprüche 1-5 * -- | 1,4-8, 11,12 | C 07 C 313/34<br>C 07 C 311/54<br>A 01 N 47/34 |
| A | DE - A1 - 3 049 440 (BAYER) * Ansprüche 1,4-7 * -- | 1,5-8 | |
| D,A | DE - A - 2 227 744 (SHELL INTERNATIONALE) * Ansprüche 1,14 * -- | 1,9 | |
| A | EP - A2 - 0 213 566 (BAYER) * Zusammenfassung * ---- | 1,4-8 | |

RECHERCHIERTE SACHGEBIETE (Int Cl⁵)

C 07 C 313/00
C 07 C 311/00

Der vorliegende Recherchenbericht wurde für alle Patentansprüche erstellt.

| Recherchenort | Abschlußdatum der Recherche | Prüfer |
|---|---|---|
| WIEN | 15-05-1991 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82